# EUROPEAN PATENT APPLICATION

(11) **EP 3 333 250 A1**
(43) Date of publication of application: **13.06.2018**
(21) Application number: 17194675.9
(22) Date of filing: 04.10.2017
(51) Int. Cl.: C12M 1/06, C12M 1/02, B01F 7/16

(54) **BIOREACTOR AND BIOGAS PRODUCTION METHOD**

(30) Priority: 01.12.2016 LT 2016530
(71) Applicant: Vilniaus Gedimino technikos universitetas, 10223 Vilnius (LT)
(72) Inventor: BALTRENAS, Pranas, 07143 Vilnius (LT); BALTRENAITE, Edita, 07143 Vilnius (LT); KOLODYNSKIJ, Vitalij, 10102 Vilnius (LT)
(74) Representative: Pranevicius, Gediminas

(57) **Abstract**

The bioreactor of the present invention characterizes by having an additional aerobic-anaerobic camera (9) for biomass treatment arranged between the primary biomass preparation camera (1) and the main anaerobic camera (15). The camera (9) is used for preparing biomass for further anaerobic digestion in the main anaerobic camera (15). The deoxidation process takes place therein, the biomass is mixed with a modified mechanical mixer (13) consisting of a surface mixer (13A), a mixer with vertical blades (13B), and spiral mixer (13C), and heated by four heating elements (12) to a temperature of 30-35°C suitable to mesophilic process. The main anaerobic camera (15) produces biogas. The camera (15) has four biomass heating elements (18) and a modified mechanical mixer (19) consisting of a surface mixer (19A), a mixer with vertical blades (19B), and a spiral mixer (19C). All mixers are arranged on a single shaft (4). The gravity movement of biomass from the camera (9) to the camera (15) is controlled by automatic valves (7B) and (7D) controlled by an automatic control unit (24) depending on signals of pressure sensors (11G) and (11K). Produced biogas is stored in a gasholder (23).

## Description

### TECHNICAL FIELD

The invention relates to biogas production devices and methods, concretely - to bioreactors, which are used for the production of high quality biogas from various organic waste.

### BACKGROUND ART

Bioreactors and biogas production method can be used practically in all catering establishments, large and small farms, poultry farms, landfills, food production companies and wastewater treatment facilities, that produce organic waste. As organic matter decomposes, large quantities of greenhouse gases (hereinafter referred to as "GHG"), such as methane CH₄ and carbon dioxide CO₂, are released into the environment. Both gaseous components absorb infrared rays, that are reflected from the ground surface. In this way, the thermal energy accumulates in the atmosphere and has negative impact on climate change. It should be noted, that a negative effect on global warming of CH₄ is 21-25 times stronger than the same molten volume of CO₂. In addition, methane is a flammable gas, which can be used as a natural gas analogue (the energy, produced by natural gas combustion is about 30% higher compared to the energy, produced by combusting the same volume
of biogas). What is more, problems of unpleasant odors spread are caused by excess sewage sludge from wastewater treatment plants, which goes to landfills and decomposes there. To be noted, odors can be removed in bioreactors. Thus, in order to reduce the negative impact on the atmosphere and obtain "green" energy for further use, it is important to use bioreactors. The bioreactor is an economical and environmentally friendly device, which reduces emissions to the air and produces biogas.

The main disadvantage of bioreactors is the high enough electricity demand, needed to maintain optimum temperature in the cameras, or by pumping organic mass from one camera to another, (e.g. from the preparation camera to the biogas production camera) by using sewage pumps. In addition, depending on the viscosity of the biomass, zones of different temperature and mixing can be formed in anaerobic camera. Methanogenic bacteria are particularly sensitive anaerobes, so even a slight temperature variation or uneven distribution can have a negative effect on the biogas production process. To solve this problem, the construction of the bioreactor must be modified.

The prototype of the present invention is a bioreactor for biogas production (Lithuanian Patent No. 5666). The device consists of two capacities: primary biomass preparation camera (upper) and main camera (lower). The cameras are installed in a single airless base. Both cameras have mechanical mixers. Biomass heating in both cameras is carried out by using heating elements, that are mounted on a sloping bottom. The space between the bioreactor vessel and the sloping bottom is filled with a heat transporting medium. The biomass is loaded through a cover to a pre-preparation camera, where biomass is being mixed and warmed up. Later, prepared for anaerobic digestion biomass is being loaded to the main camera. It should be noted, that the movement of biomass between cameras in this invention is carried out under gravitational forces. The biogas production process is controlled by an automatic control unit.

The described prototype has several drawbacks. At the first, the primary preparation camera is opened every time it is desired to fill it. In this way, the excess air enters the camera. To optimize biomass, i.e. to remove excess air, which later has negative impact on process in anaerobic camera, it is necessary to maintain the biomass in the preparation camera for a longer period of time, which increases the energy consumption (while heating and mixing of biomass). Also, the invention provides biomass heating using heating elements in both cameras that are mounted on a sloping bottom. By heating the biomass in this way, different temperature zones are formed, i.e. the temperature of biomass in the bottom of the camera will be higher than in the central or upper parts. The more viscous the biomass is, the greater temperature differences will be, which are problematic to remove by mixing. It has been observed, that the mechanical mixers, used in the invention, do not ensure complete mixing of viscous biomass. In addition, in the central part of surface of biomass, a film can form, which prevents the release of biogas. The design of the main camera cover is flat, so bacteria, which are present in water steam during biogas production process, can die after impact on a rectangular structure, and therefore it is better to apply a convex cover of the camera. Also, in each camera separate mechanical mixers, that have a separate electric motor, are used. This constructional decision doubles the demand of electricity. Thus, in order to optimize the process, it is necessary to modify the design of the prototype and the biogas production method.

### SUMMARY OF THE INVENTION

The purpose of the invention is to improve the design of a bioreactor and the biogas production method, in order to reduce electricity consumption, to use organic waste more efficiently in the biogas production process, and, at the same time, to reduce the negative impact on the environment.

The design and method of an improved bioreactor provides an opportunity to optimize the biogas production process. The biogas production method can be divided into 3 main stages: 1) biomass preparation; 2) optimization of biomass parameters in an aerobic-anaerobic camera; 3) production of biogas in an anaerobic camera. The bioreactor is designed to meet the basic engineering principles: efficiency, economy and reliability. These principles are achieved through a number of solutions. Only one electric motor is used for biomass mixing, i.e. the biomass is mixed simultaneously in all cameras. The invention provides three cameras, the joint work of which is aimed at increasing the output and quality of biogas. The preparation camera with a large fraction organic waste crusher, in which the primary mixing of the biomass takes place, is separated from the aerobic-anaerobic camera by biomass supply channel, because the excess oxygen amount enters the camera each time the biomass is supplied. It should be noted, that 2/3 of the biomass remains in the aerobic-anaerobic camera, and it closes the way for oxygen to enter. By using this constructive solution, the aerobic-anaerobic camera does not contain excess oxygen. An aerobic-anaerobic camera implements a transitional process when the biomass from an aerobic (saturated in dissolved oxygen biomass) state passes to anaerobic (anoxic) state. To achieve this, the biomass is mixed by the modified mixer and heated by four heating elements, located between the vessel of the camera and the vertical blades of the mixer. It should be noted that the purpose of this camera is directly related to microbiological processes in the main (anaerobic) camera in which the biogas is produced. The methane fermentation process is carried out by two main groups of microorganisms (acidic and methanogenic bacteria) in three stages: hydrolysis, oxidation and methane production. Other groups of microorganisms are involved in the process too, but the main group is the methanogenic bacteria that belong to the anaerobic group, which is particularly sensitive to changes in temperature and oxygen concentrations (a sudden increase of oxygen concentration during anaerobic digestion decreases the efficiency of the methane production process). In the different temperatures and mixing of individual biomass zones, the biogas production process can significantly decrease qualitatively and quantitatively. Because of this, biomass has to be kept in an aerobic-anaerobic camera for some time, in which it is thoroughly mixed and warmed to a temperature favorable for the bacteria. Since the camera is closed, deoxidation processes are taking place, the organic material is fermented by extracellular enzymes (cellulose, amylase, protease and lipase). Bacteria destroy long complex hydrocarbons such as proteins and lipids, and generate short chains. Acidic bacteria, that are involved in the second stage of biogas production process, break down complex organic compounds (fiber, protein, fat, etc.) into simpler ones. As a result, the initial fermentation products - volatile fatty acids, hydrogen, carbon monoxide, acetic acid, formic acid, etc. appear in the biomass. Methanogenic bacteria, which convert organic acids into biogas, use these organic materials as a source of food. As a result, small colonies of acidic and methanogenic bacteria appear, which interact symbiotically and whose colonies will increase after entering the anaerobic camera. In this way, deoxidized, prepared for the production of biogas and supplied into an anaerobic camera, biomass does not negatively affect the other bacterial colonies, which are already present in the anaerobic camera.

The vessel of the anaerobic camera is modified, its cover is convex, because when water is steamed or biogas is released, there is also a certain amount of bacteria that die after impact on a flat cover. In the case of a convex cover, vortices are formed and the negative impact on bacteria is reduced. In optimum case, the radius of curvature must correspond to the radius of an anaerobic camera of the bioreactor. In order to increase the yield of biogas, mechanical aerobic-anaerobic and anaerobic camera mixers were modified. Biomass in the cameras is mixed using a surface mixer (to remove film that blocks the biogas yield), vertical mixer blades (to mix biomass in the area near the vessel of the bioreactor, from the bottom of the anaerobic camera to the biomass surface), spiral mixer (to mix the central part of the biomass over the entire height of the anaerobic camera). The vertical blade angle α is 45° to reduce the pressure resistance of blades to mixing biomass and increase the biomass mixing efficiency. By applying such a mixing method, the biomass is maintained in its homogeneous state over its entire volume, i.e. even with high biomass viscosity, denser and different in temperature zones does not appear in the camera, and no film forms on the surface, that reduce the effectiveness of the biogas production process. Vertical heating elements are used to heat biomass. The elements are located between the vessel of the camera and the blades of the vertical mixer. Such complex mixing and heating throughout the entire height of the camera ensures an even distribution of temperature and mix throughout the operation volume of the camera. The movement of biomass from one camera to another is controlled by automatic valves, whose operation is controlled by an automatic control unit. The bottom of all cameras is sloping in order to ensure the full supply of biomass from one camera to another.

### DESCRIPTION OF THE FIGURES OF DRAWINGS

Fig. 1 is a schematic view of a bioreactor in longitudinal section;
Fig. 2 is a cross section of a bioreactor's anaerobic camera.

### DETAILED DESCRIPTION OF THE INVENTION

The bioreactor consists of: 1 - biomass preparation camera; 2 - cover of the biomass preparation camera; 3 - electromotor; 4 - mixer shaft; 5 - mechanical mixer of the biomass preparation camera; 6 - mixed biomass supply channel; 7B - automatic valve of the preparation camera; 7D - automatic valve of the aerobic-anaerobic camera; 7E - automatic valve of the anaerobic camera; 8 - support; 9 - aerobic-anaerobic camera; 10 - excess pressure valve; 11G - pressure sensor of the aerobic-anaerobic camera; 11K - pressure sensor of the anaerobic camera; 12 - heating element; 13 - modified aerobic-anaerobic camera mixer; 13A - surface mixer; 13B - mixer with vertical blades; 13C - spiral mixer; 14 - optimized biomass supply channel; 15 - anaerobic camera; 16 - convex cover of the anaerobic camera; 17 - biogas output; 18 - heating element; 19 - modified anaerobic camera mixer; 19A - surface mixer; 19B - mixer with vertical blades; 19C - spiral mixer; 20 - fermented biomass removal channel; 21 - thermal insulation layer; 22 - biogas supply channel; 23 - gasholder; 24 - automatic control unit; 25 - waste crusher.

### Device operation principle

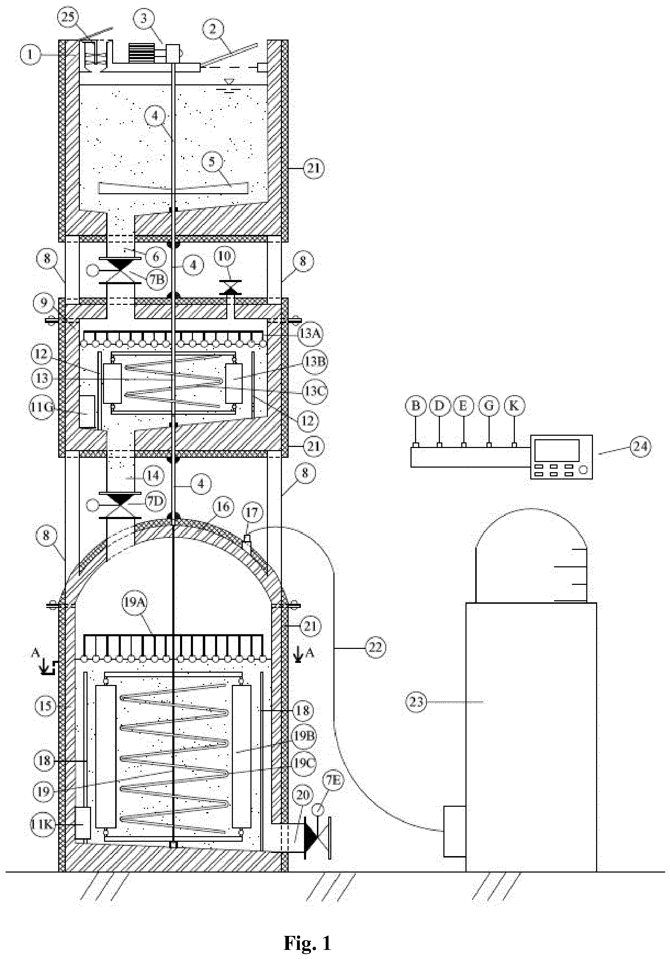

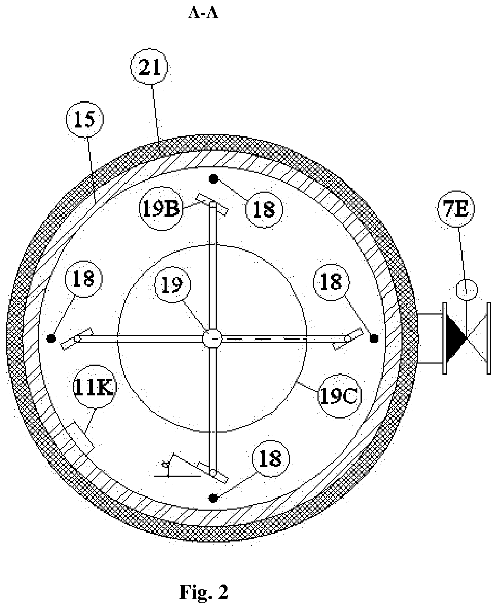

The principle of the operation of the bioreactor is given in Figures 1 and 2. Biomass is prepared in the preparation camera 1. A small fraction or liquid organic material is supplied through the camera cover 2. In the case of large fractions of organic waste, a waste crusher 25 is used. All bioreactor camera mixers operate by using a single electric motor 3. The mixing elements are locked onto a common shaft 4. Mechanical mixer 5 of preparation camera is used for organic mass mixing, until homogeneous biomass is obtained. Part of prepared biomass (10% of the anaerobic camera's 15 volume) is supplied to aerobic-anaerobic camera 9 by biomass supply channel 6. Biomass moves under gravity forces and its movement is controlled by an automatic valve 7B. The aerobic-anaerobic camera 9 is designed to increase the biomass temperature to the temperature of the mesophilic process (up to 30-35°C), remove the different mixing zones and reduce the oxygen concentration. Camera 9 is always filled with biomass, which is being prepared for methane production process. Its maximum operating volume reaches 30% of the anaerobic camera's 15 volume. It should be noted that the minimum filling of aerobic-anaerobic camera 9 amounts to 20% of the anaerobic camera's 15 volume. The disposable supply of biomass to the aerobic-anaerobic camera 9 or the discharge from it reaches 10% of the anaerobic camera's 15 volume. Such a decision is based on the following facts: 1) it has been experimentally determined, that without the negative effect on the methane production process, no more than 10% of the anaerobic camera's 15 volume of the degased biomass can be removed from the anaerobic camera 15, and accordingly no more than 10% of prepared biomass of the anaerobic camera's 15 volume can be supplied from aerobic-anaerobic camera 9; 2) the size of the aerobic-anaerobic camera 9 is selected based on the above-described point and in order to reduce the energy consumption by mixing and heating the biomass in the aerobic-anaerobic camera 9, because when supplying the biomass to the camera 9 from the preparation camera 1, the camera 9 is already filled up to 2/3 by the operating volume. This means that a new biomass (1/3 of the of the camera's 9 volume) is mixed with the previously mixed and heated biomass (2/3 of the camera's 9 volume).

After supplying biomass (10% of the anaerobic camera's 15 volume) from an aerobic-anaerobic camera 9 to an anaerobic camera 15, the same volume of biomass is supplied from a preparation camera 1 to an aerobic-anaerobic camera 9. The operating volume of an aerobic-anaerobic camera 9 at this stage reaches 30% of the anaerobic camera's 15 volume. In this way, the newly prepared in the aerobic-anaerobic camera 9 biomass can be prepared for further usage much quicker than if the case when entire volume of the aerobic-anaerobic camera 9 is periodically supplied to the anaerobic camera 15. So, the continuous preparation of biomass in the aerobic-anaerobic camera 9 and the methane production process in an anaerobic camera 15 are cyclically carried out. All bioreactor cameras are arranged one above the other and fixed with supports 8. An excessive air pressure generates when biomass is supplied to aerobic-anaerobic camera 9. This pressure prevents biomass movement from one camera to another, so the camera 9 has an excess pressure valve 10. For some time, the biomass is heated using four heating elements 12 which are arranged between the vessel of the camera 9 and the vertical blades 13B of the mixer 13. The biomass is mixed by using a modified aerobic-anaerobic camera 9 mixer 13 consisting of three structural elements: surface mixers 13A, mixers with vertical blades 13B (with an angle α of 45°) and spiral mixers 13C. The biomass level in the camera 9 is controlled by a pressure sensor 11G. The biomass, prepared in the camera 9, is supplied to the anaerobic camera 15 by optimized biomass supply channel 14. The biogas production process in an anaerobic camera 15 is controlled in the following order:
1) a part, i.e. 10% of the anaerobic camera's 15 volume, of degassed biomass is removed from an anaerobic camera 15 with an automatic valve 7E. The valve is controlled by the automatic control unit 24 (receiver E). The valve 7E automatically opens after a certain time which is set by the user of the bioreactor, depending on the amount of organic waste to be recycled;
2) when the biomass level in the anaerobic camera 15 decreases, the pressure, which is controlled by a pressure sensor 11K, is reduced. A signal is sent to the automatic control unit 24 (receiver K). The valve 7D (automatic control unit 24 receiver D) opens and the heated and mixed biomass (10% of the anaerobic camera's 15 volume) is supplied from the aerobic-anaerobic camera 9 to the anaerobic camera 15. Filling of the aerobic-anaerobic camera 9 at this stage reaches 20% of the anaerobic camera's 15 volume (total volume of the aerobic-anaerobic camera 9 reaches 30% of volume of the anaerobic camera 15).
3) after biomass is optimized in the aerobic-anaerobic camera 9, it is supplied to the anaerobic camera 15. A pressure sensor 11G sends a signal to the automatic control unit 24 (receiver G) and the valve 7B (automatic control unit 24 receiver B) is opened. Biomass (10% of the anaerobic camera's 15 volume) is supplied from preparation camera 1 to the aerobic-anaerobic camera 9. Filling of the aerobic-anaerobic camera 9 at this stage reaches 30% of the anaerobic camera's 15 volume (the total volume of the aerobic-anaerobic camera 9 reaches 30% of the anaerobic camera's 15 volume);
4) the biomass is heated, mixed, deoxygenated, and the process is repeated.

The anaerobic camera 15 has a convex cover 16 and four heating elements 18 that maintain the temperature required for the mesophilic process. Elements 18 are arranged between the anaerobic camera 15 vessel and the vertical blades 19B of the mixer 19. The biomass is periodically mixed with a modified mechanical mixer 19. The mixer 19 consists of three mechanical elements: 1) an surface mixer 19A that is intended to remove the film on the surface of the biomass so that it does not block the yield of biogas; 2) vertical blades 19B; 3) spiral mixer 19C. Such complex mixing of biomass allows elimination of uneven distribution of temperature and different mixing of biomass in all zones of the camera 15. The blade angle α of the mixer 19B is 45° to reduce the pressure resistance during mixing. The part of degazed biomass is disposed through the fermented biomass removal channel 20. Produced biogas moves through biogas output 17 and supplied to gasholder 23 for further use by biogas supply channel 22. For the reduction of heat loss, the bodies of the biomass preparation camera 1, aerobic-anaerobic camera 9 and anaerobic camera 15 must be insulated with a thermal insulation layer 21.

## Claims

1. A bioreactor containing a primary biomass preparation camera with a biomass mixer, sloping bottom for biomass movement downward under gravity, and biomass heating elements, wherein the biomass is mixed up to a homogeneous consistency, and a main anaerobic camera with biomass mixer and biomass heating elements, located below the primary camera, wherein biomass fermentation takes place and biogas is generated, **characterized by** having:
- an additional aerobic-anaerobic biomass treatment camera (9), arranged between the primary biomass preparation camera (1) and the main anaerobic camera (15), designed to increase the biomass temperature to the temperature of the mesophilic process (up to 30-35°C), to remove different mixing zones and to reduce the oxygen concentration in biomass;
- a main anaerobic camera (15) with a convex cover (16) for the production of biogas;
- a single shaft (4) controlled by a single electric motor (3), which runs vertically through all three - biomass preparation (1), the additional aerobic-anaerobic (9) and the main anaerobic (15) - cameras, which is equipped with separate modified biomass mixers (13) and (19) for mixing biomass in cameras (9) and (15);
- biomass heating elements (12) and (18) installed in cameras (9) and (15) between the mixers (13) and (19) and the inner walls of the cameras (9) and (15) over the entire height of the heated biomass;
- pressure sensors (11G) and (11K) arranged in cameras (9) and (15), where the pressure sensor (11K) serves to control the supply of homogenized biomass from the additional aerobic-anaerobic camera (9) to the main anaerobic camera (15), and the pressure sensor (11G) serves to control the supply of homogenized biomass from the primary biomass preparation camera (1) into an additional aerobic-anaerobic camera (9).

2. The bioreactor according to claim 1, **characterized in that** the radius of the convex cover (16) of the main anaerobic camera (15) is equal to the radius of the cross section of the anaerobic camera (15).

3. The bioreactor according to claim 1, **characterized in that** the biomass mixer (13) of the additional aerobic-anaerobic camera (9) consists of three structural elements: surface mixer (13A), mixer with vertical blades (13B), and spiral mixer (13C).

4. The bioreactor according to claim 3, **characterized in that** the vertical blades (13B) of the biomass mixer (13) are rotated at an angle α to reduce the pressure resistance of the blades (13B) to the mixing biomass and increase the biomass mixing efficiency.

5. The bioreactor according to claim 4, **characterized in that** the angle α is equal to 45°.

6. The bioreactor according to claim 1, **characterized in that** the biomass mixer (19) of the main anaerobic camera (15) consists of three structural elements: surface mixer (19A), mixer with vertical blades (19B), and spiral mixer (19C).

7. The bioreactor according to claim 6, **characterized in that** the vertical blades (19B) of the biomass mixer (19) are rotated at an angle α to reduce the pressure resistance of the blades (19B) to the mixing biomass and increase the biomass mixing efficiency.

8. The bioreactor according to claim 7, **characterized in that** the angle α is equal to 45°.

9. The bioreactor according to claim 1, **characterized by** having at least four biomass heating elements (12) arranged within the inner circumference of the additional aerobic-anaerobic camera (9) between the mixer (13) and the inner wall of the camera (9) over the entire height of the heated biomass.

10. The bioreactor according to claim 1, **characterized by** having at least four biomass heating elements (18) arranged within the inner circumference of the main anaerobic camera (15) between the mixer (19) and the inner wall of the camera (15) over the entire height of the heated biomass.

11. The bioreactor according to claim 1, **characterized in that** the biomass preparation camera (1) and the additional aerobic-anaerobic camera (9) are interconnected by the biomass supply channel (6), which is controlled by an automatic valve (7B).

12. The bioreactor according to claim 1, **characterized in that** the additional aerobic-anaerobic camera (9) and the main anaerobic camera (15) are interconnected by a biomass supply channel (14) which is controlled by an automatic valve (7D).

13. The bioreactor according to claim 1, **characterized in that** the volume of the additional aerobic-anaerobic camera (9) reaches 30% of the volume of the main anaerobic camera (15).

14. The bioreactor according to claim 1, **characterized by** having supports (8) for fixing all three - biomass preparation (1), additional aerobic-anaerobic (9) and main anaerobic (15) - bioreactor's cameras located vertically one above the other.

15. The bioreactor according to claim 1, **characterized by** having a valve (10) for discharging the excess pressure of the air generated in the additional camera (9).

16. A biogas production method for anaerobic production of methane from organic waste biomass, **characterized in that**
- the biomass, being crushed and mixed to a homogeneous consistency in the primary biomass preparation camera (1), is further treated in an additional aerobic-anaerobic camera (9), wherein the biomass is thoroughly mixed, warmed to a temperature of 30-35°C favorable to methanogenic bacteria, and deoxidized, i.e. it goes from an aerobic state to anaerobic state;
- 10% of the fermented biomass, used for the production of biogas in the main anaerobic camera (15), is removed gravitationally through the channel (20);
- 30% of biomass prepared prepared in the additional aerobic-anaerobic camera (9) is gravitationally supplied through the channel (14) to the main anaerobic camera (15);
- the additional aerobic-anaerobic camera (9) being supplied with a new identical volume of biomass from the primary biomass preparation camera (1) through the channel (6), thereby ensuring the cyclic and continuous operation of the bioreactor.

17. The method according to claim 16, **characterized by** activating the removal of the fermented biomass from the main anaerobic camera (15) through the channel (20), after receiving a signal from the control unit (24) which opens the valve (7E) of the channel (20).

18. The method according to claim 16, **characterized in that** the control unit (24) activates the supplying of biomass heated to a temperature of 30-35 °C and deoxidized from an additional anaerobic-anaerobic camera (9) to the main anaerobic camera (15) by opening the valve (7D) of the channel (14) after having received a pressure sensor's (11K) signal about the reduced pressure and, consequently, the biomass level in the main anaerobic camera (15).

19. The method according to claim 16, **characterized in that** the control unit (24) activates the supplying of a biomass from primary preparation camera (1) to the additional aerobic-anaerobic camera (9) by opening the valve (7B) of the channel (6) after having received a pressure sensor's (11G) signal about the reduced pressure and, consequently, the biomass level in the additional aerobic-anaerobic camera (9).

20. The method according to claim 16, **characterized by** using the modified mixers (13) and (19), which are installed in cameras (9) and (15) and which consist of surface mixers (13A) and (19A), mixers with vertical blades (13B) and (19B), and spiral mixers (13C) and (19C), which ensure a thermally uniform and homogeneous mixing of the biomass over the entire volume in cameras (9) and (15).
